# EUROPEAN PATENT APPLICATION

(11) **EP 4 530 974 A1**
(43) Date of publication of application: **02.04.2025**
(21) Application number: 23306619.0
(22) Date of filing: 27.09.2023
(51) Int. Cl.: G06T 7/00, G06T 5/50

(54) **OCCLUDED VESSEL VISUALIZATION**

(71) Applicant: Koninklijke Philips N.V., 5656 AG Eindhoven (NL)
(72) Inventor: FLORENT, Raoul, 5656 EINDHOVEN (NL); HENDRIKS, Bernardus, 5656 EINDHOVEN (NL); VAN MOURIK, Martijn, 5656 EINDHOVEN (NL); LAI, Marco, 5656 EINDHOVEN (NL); LUCASSEN, Gerald, 5656 EINDHOVEN (NL); SPLIETHOFF, Jarich, 5656 EINDHOVEN (NL); SIO, Charles, 5656 EINDHOVEN (NL); GROEN, Joanneke, 5656 EINDHOVEN (NL); LARUE, Ruben, 5656 EINDHOVEN (NL)
(74) Representative: Philips Intellectual Property & Standards

(57) **Abstract**

A system for providing an image representation of an occluded vessel, is provided. The system comprising one or more processors configured to: detect a portion (140ₚ, 140_{d}) of the vessel abutting the occlusion (130) in a sequence of angiographic images (120); extract, from the sequence of angiographic images (120), motion vectors (150) representing a motion of the portion (140ₚ, 140_{d}) of the vessel, based on the detected portion of the vessel; mutually register the sequence of angiographic images (120) based on the extracted motion vectors (150); process the image intensities in the mutually-registered sequence of angiographic images in a region of interest adjoining the portion (140ₚ, 140_{d}) of the vessel to provide the image representation of the occluded vessel; and output the image representation of the occluded vessel.

## Description

### TECHNICAL FIELD

The present disclosure relates to providing an image representation of an occluded vessel. A system, a computer-implemented method, and a computer program product, are disclosed.

### BACKGROUND

Over time, blood vessels may become obstructed, or in other words, occluded. Occlusions can occur for various reasons, including as a result of the build-up of plaque, or the development of a clot, in the vessel. Occlusions can occur in blood vessels in various parts of the anatomy, including in the heart, the brain, the lung, the leg, and so forth.

In some cases an occlusion is partial, and blood flow in the vessel is restricted, but not blocked. For instance, in the heart, a partial occlusion of a coronary artery may result in the heart having to work harder in order to maintain blood flow. However, in more severe cases, blood flow in a vessel may be completely blocked by an occlusion. A chronic total occlusion, or "CTO" is defined as a complete blockage of a blood vessel for a duration of greater than or equal to 3 months.

If a vessel becomes occluded, a treatment procedure may be required. An example of such a treatment procedure is a balloon angioplasty procedure. A balloon angioplasty procedure has the effect of opening-up a vessel lumen, thereby restoring blood flow through the vessel. Various interventional devices may be used in such treatment procedures, including a guidewire, a (balloon) catheter, and so forth. A balloon angioplasty often includes the placement of a stent in the vessel, and which serves to maintain the vessel lumen in the opened-up state
Medical investigations relating to occluded vessels are often performed using angiographic images. X-ray angiographic images, i.e. X-ray images that are acquired subsequent to the injection of a contrast agent into the vasculature, are often used for this purpose. The contrast agent introduces a difference between the X-ray attenuation of blood, and surrounding tissue, thereby improving the contrast between the vessel lumen, and the surrounding tissue in the X-ray angiographic images. However, the limited amount, or in the case of a CTO, the absence, of blood, and consequently contrast agent in the occlusion itself results in poor visibility of occlusions in X-ray angiographic images. Occluded vessels may therefore only appear in angiographic images as faint image features arising from the attenuation of vessel walls, calcifications, and any contrast agent that manages to pass along the vessel lumen. The limited amount, or absence, of blood in an occlusion also results in the poor visibility of occlusions in angiographic images that are generated using other types of imaging modalities. This presents challenges when investigating, planning, and also executing, treatment procedures relating to occlusions.

Consequently, there is a need to improve the visualization of occluded vessels.

### SUMMARY

According to one aspect of the present disclosure, a system for providing an image representation of an occluded vessel, is provided. The system comprises one or more processors configured to:
receive image data representing a sequence of angiographic images comprising an occlusion in a vessel;
detect a portion of the vessel abutting the occlusion in the sequence of angiographic images;
extract, from the sequence of angiographic images, motion vectors representing a motion of the portion of the vessel, based on the detected portion of the vessel;
mutually register the sequence of angiographic images based on the extracted motion vectors;
process the image intensities in the mutually-registered sequence of angiographic images in a region of interest adjoining the portion of the vessel to provide the image representation of the occluded vessel; and
output the image representation of the occluded vessel.

In comparison to non-occluded vessels, occluded vessels are typically only poorly visible in angiographic images. In the above system, angiographic images are mutually registered using motion vectors that are determined based on the motion of a detected portion of a vessel abutting the occlusion. Using the portion of the vessel abutting the occlusion to mutually-register the images ensures that the images are accurately registered in the vicinity of the occlusion. This portion of the vessel is also visible in angiographic images because blood reaches this portion of the vessel. The image intensities in the mutually-registered sequence of angiographic images are then processed in a region of interest adjoining the portion of the vessel to provide an image representation of the occluded vessel. The image intensities may be processed using techniques such as temporal integration, for example. The image representation may be referred to as an enhanced image representation of the occluded vessel since the visibility of the occluded vessel is enhanced as compared to in the sequence of angiographic images. Since this processing is based on the accurately co-registered images, faint image features of the occluded vessel arising from the attenuation of vessel walls, calcifications, and any contrast agent that manages to pass along the vessel lumen, are well-aligned in the region of interest in the mutually-registered images. This facilitates their processing so as to provide an accurate image representation of the occluded vessel.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for providing an image representation of an occluded vessel, in accordance with some aspects of the present disclosure.
Fig. 2 is a flowchart illustrating an example of a computer-implemented method of providing an image representation of an occluded vessel, in accordance with some aspects of the present disclosure.
Fig. 3 illustrates an example of a sequence of angiographic images 120 comprising an occlusion 130 in a vessel 140, in accordance with some aspects of the present disclosure.
Fig. 4 illustrates an example of an angiographic image including detected portions 140ₚ and 140_{d} of a vessel abutting an occlusion 130, in accordance with some aspects of the present disclosure.
Fig. 5 is a schematic illustration of an example of the extraction of motion vectors 150 representing the motion of portions 140ₚ and 140_{d} of a vessel abutting an occlusion 130, in accordance with some aspects of the present disclosure.
Fig. 6 is a first example of an image representation 160 of an occluded vessel including an extension to the portion of the vessel abutting the occlusion towards the occlusion, in accordance with some aspects of the present disclosure.
Fig. 7 is a second example of an image representation 160 of an occluded vessel including an extension to the portion of the vessel abutting the occlusion towards the occlusion, in accordance with some aspects of the present disclosure.
Fig. 8 is a third example of an image representation 160 of an occluded vessel including an extension to the portion of the vessel abutting the occlusion towards the occlusion, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of a system for providing an image representation of an occluded vessel. In some examples, the occluded vessel is a coronary artery, i.e. a blood vessel located in the heart. It is, however, to be appreciated that the coronary artery, and the heart, serve only as examples of a location, and a type, of the occluded vessel. In general, the occluded vessel may be any type of blood vessel, including an artery, or a vein, for example. In general, the occluded vessel may be in any location in the anatomy, including in the heart, the brain, the lung, the leg, and so forth. Reference is also to made herein to examples in which the occlusion is a CTO. It is, however, to be appreciated that the occlusion may in general be any type of occlusion. For instance, the occlusion may alternatively be a partial occlusion.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being are performed by the one or more processors of the system disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there is a need to improve the visualization of occluded vessels.

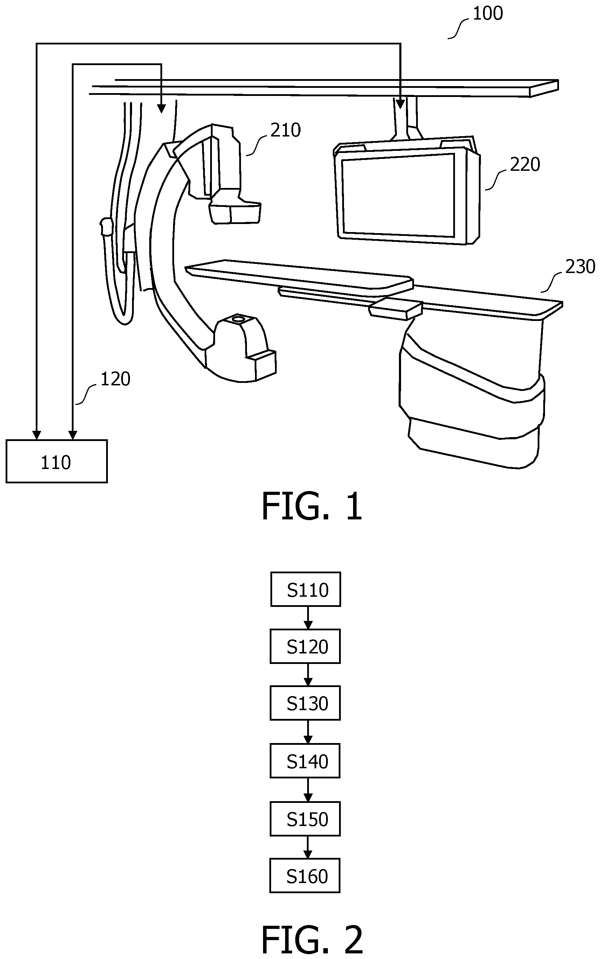

Fig. 1 is a schematic diagram illustrating an example of a system 100 for providing an image representation of an occluded vessel, in accordance with some aspects of the present disclosure. Fig. 2 is a flowchart illustrating an example of a computer-implemented method of providing an image representation of an occluded vessel, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1.

With reference to Fig. 1, and Fig. 2, the system 100 for providing an image representation of an occluded vessel comprises one or more processors 110 configured to:
receive S110 image data representing a sequence of angiographic images 120 comprising an occlusion 130 in a vessel 140;
detect S120 a portion 140ₚ, 140_{d} of the vessel abutting the occlusion 130 in the sequence of angiographic images 120;
extract S130, from the sequence of angiographic images 120, motion vectors 150 representing a motion of the portion 140ₚ, 140_{d} of the vessel, based on the detected portion of the vessel;
mutually register S140 the sequence of angiographic images 120 based on the extracted motion vectors 150;
process S150 the image intensities in the mutually-registered sequence of angiographic images in a region of interest adjoining the portion 140ₚ, 140_{d} of the vessel to provide the image representation 160 of the occluded vessel; and
output S160 the image representation 160 of the occluded vessel.

As mentioned above, in comparison to non-occluded vessels, occluded vessels are typically only poorly visible in angiographic images. In the above system, angiographic images are mutually registered using motion vectors that are determined based on the motion of a detected portion of a vessel abutting the occlusion. Using the portion of the vessel abutting the occlusion to mutually-register the images ensures that the images are accurately registered in the vicinity of the occlusion. This portion of the vessel is also visible in angiographic images because blood reaches this portion of the vessel. The image intensities in the mutually-registered sequence of angiographic images are then processed in a region of interest adjoining the portion of the vessel to provide an image representation of the occluded vessel. The image intensities may be processed using techniques such as temporal integration, for example. The image representation may be referred to as an enhanced image representation of the occluded vessel since the visibility of the occluded vessel is enhanced as compared to in the sequence of angiographic images. Since this processing is based on the accurately co-registered images, faint image features of the occluded vessel arising from the attenuation of vessel walls, calcifications, and any contrast agent that manages to pass along the vessel lumen, are well-aligned in the region of interest in the mutually-registered images. This facilitates their processing so as to provide an accurate image representation of the occluded vessel.

The operations performed by the system 100 are described in more detail below.

In the operation S110, image data is received. The image data represents a sequence of angiographic images 120 comprising an occlusion 130 in a vessel 140.

The sequence of angiographic images 120 that is received in the operation S110 may be generated by various types of imaging systems. These include two-dimensional imaging systems such as projection X-ray imaging systems, and also volumetric imaging systems such as computed tomography "CT" imaging systems and magnetic resonance imaging "MRI" systems. In examples in which the sequence of angiographic images 120 is generated by a projection X-ray imaging system, or by a CT imaging system, the imaging system may be a spectral X-ray projection imaging system, or a spectral CT imaging system, respectively. Such imaging systems generate X-ray attenuation data representing X-ray attenuation within multiple different energy intervals. The X-ray attenuation data generated by such imaging systems may be processed, e.g. using various material decomposition algorithms, in order to distinguish between media that have similar X-ray attenuation values when measured within a single energy interval, and which would be indistinguishable in X-ray attenuation data obtained using conventional projection X-ray imaging systems, or conventional CT imaging systems. Thus, X-ray attenuation data generated by spectral X-ray projection imaging systems, or by spectral CT imaging systems, may be processed to provide angiographic images with improved specificity to materials such as contrast agent, tissue, bone, and so forth.

With continued reference to the operation S110, in some examples, the sequence of angiographic images 120 is generated subsequent to the injection of a contrast agent into the vasculature of a subject. The contrast agent serves to improve the contrast between blood and surrounding tissue in the angiographic images 120. In these examples, the angiographic images 120 may be referred-to as contrast-enhanced angiographic images. Sequences of contrast-enhanced angiographic images may be generated by a projection X-ray imaging system, or by a CT imaging system, or by an MRI system. MRI angiographic images may alternatively be generated in the absence of a contrast agent, and are often referred-to as non-contrast-enhanced MR angiography "NC-MRA" images.

With continued reference to the operation S110, in one example, the images in the sequence of angiographic images 120 are generated instantaneously before being received by the one or more processors 110. In other words, the sequence of angiographic images 120 may be a live sequence of angiographic images 120. Alternatively, the sequence of angiographic images 120 may have been generated some seconds, minutes, hours, or even days, or a longer period, before being received by the one or more processors 110. In the latter case, the sequence of angiographic images 120 may have been generated prior to performing a medical procedure on the anatomical region. In this case, the sequence of angiographic images 120 may be referred-to as a pre-procedural sequence of angiographic images 120.

With continued reference to the operation S110, the sequence of angiographic images 120 may be received from various sources in the operation S110, including from a medical imaging system such as one of the imaging systems described above. Alternatively, the sequence of angiographic images 120 may be received from another source, such as from a computer readable storage medium, or from the internet, or from the Cloud, and so forth. In general, the sequence of angiographic images 120 may be received via any form of data communication, including wired and wireless communication. By way of some examples, when wired communication is used, the communication may take place via an electrical or optical cable, and when wireless communication is used, the communication may for example take place via RF or infrared signals.

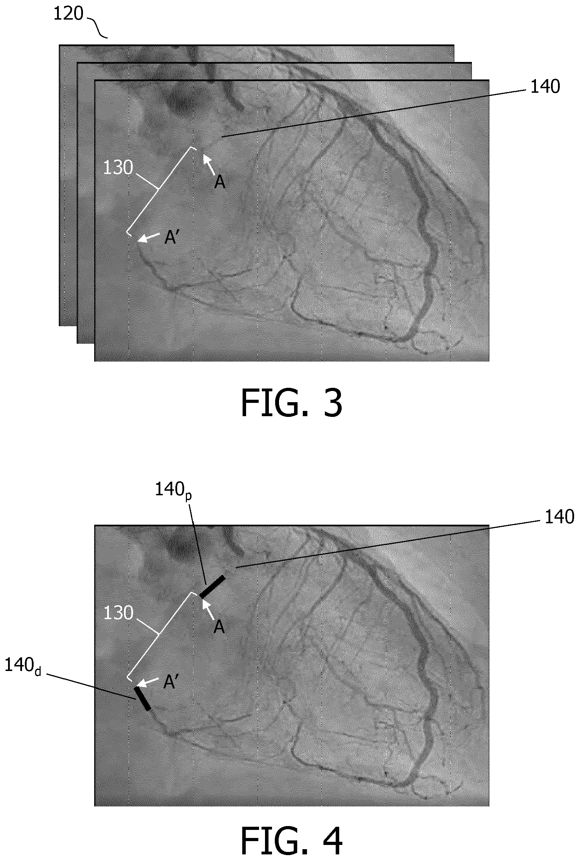

Fig. 3 illustrates an example of a sequence of angiographic images 120 comprising an occlusion 130 in a vessel 140, in accordance with some aspects of the present disclosure. The angiographic images 120 illustrated in Fig. 3 represent the vasculature of the heart. The angiographic images 120 illustrated in Fig. 3 are contrast-enhanced X-ray angiographic images that have been generated subsequent to the injection of a contrast agent into a subject. The contrast-enhanced X-ray angiographic images illustrated in Fig. 3 may be generated using a projection X-ray imaging system, such as the C-arm-based projection X-ray imaging system 210 illustrated in Fig. 1. In Fig. 3, the vessel 140 includes an occlusion 130. The occlusion 130 extends between the points labelled A, and A'. In this example, the occlusion completely blocks the flow of blood, and the occlusion 130 is a CTO. As a result of the absence of blood, and hence contrast agent, along the length of the occlusion 130, the occlusion 130 is poorly visible in the angiographic images 120.

Referring now to the operation S120 mentioned above with reference to Fig. 1 and Fig. 2; in this operation, a portion 140ₚ, 140_{d} of the vessel abutting the occlusion 130 is detected in the sequence of angiographic images 120. As mentioned above with reference Fig. 3, whilst the occlusion between the points labelled A, and A', in the vessel 140 is typically poorly visible due to the absence of contrast agent along the occluded part of the vessel, a portion of the vessel abutting the occlusion is visible in the angiographic images because blood reaches this portion of the vessel. This portion of the vessel 140 may be referred-to as a "stump" of the vessel because beyond this portion of the vessel, the vessel appears to be truncated in the angiographic images 120.

In the example illustrated in Fig. 3, two portions of the vessel abutting the occlusion 130 are visible: a proximal portion terminating at point A, and a distal portion beginning at point A'. The terms "proximal" and "distal" in this context refer to relative positions with respect to the direction of conventional blood flow in the vessel. In general, a single, i.e. a proximal or a distal, or alternatively both, i.e. a proximal and a distal, portion of the vessel 140 abutting the occlusion 130 may be detected in the operation S120. The portion of the vessel that is detected in the operation S120 may be a point, such as a distal, or proximal, end of the vessel abutting the occlusion 130. Alternatively it may be a longer portion of the vessel, such as a segment of the vessel.

Fig. 4 illustrates an example of an angiographic image including detected portions 140ₚ and 140_{d} of a vessel abutting an occlusion 130, in accordance with some aspects of the present disclosure. In this example, two portions of the vessel are detected, i.e. proximal portion 140ₚ and the distal portion 140_{d}. In this example, the detected portions 140ₚ and 140_{d} represent segments of the vessel abutting the occlusion 130.

Various techniques are contemplated for the detection of the portions 140ₚ, 140_{d} of the vessel abutting the occlusion in the operation S120. These include segmentation techniques, and also feature detection techniques. One example of a segmentation technique that may be used for this purpose is described in a document by Isensee, F., et al., "Automated Design of Deep Learning Methods for Biomedical Image Segmentation", pages 1-55, https://arxiv.org/pdf/1904.08128.pdf.

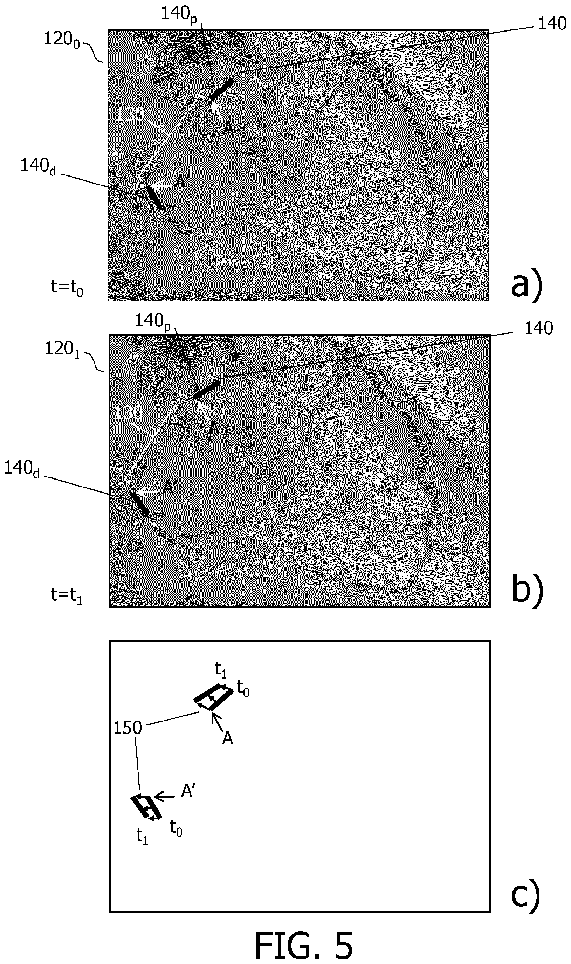

Referring now to the operation S130 mentioned above with reference to Fig. 1 and Fig. 2; in this operation, motion vectors 150 representing a motion of the portion 140ₚ, 140_{d} of the vessel, are extracted from the sequence of angiographic images 120, based on the detected portion of the vessel. This operation is described with reference to Fig. 5, which is a schematic illustration of an example of the extraction of motion vectors 150 representing the motion of portions 140ₚ and 140_{d} of a vessel abutting an occlusion 130, in accordance with some aspects of the present disclosure. Fig. 5a, and Fig. 5b illustrate angiographic images 120o and 120₁ comprising the occlusion 130 in the vessel 140 at times t = to, and t = t₁, respectively. The detected portions 140ₚ and 140_{d} of the vessel 140 abutting the occlusion that have been determined in the previous operation S120 are illustrated in each of the images in Fig. 5a, and Fig. 5b. Due to cardiac motion, the positions of the detected portions 140ₚ and 140_{d} of the vessel 140, change between the times t = to, and t = t₁. This is illustrated in Fig. 5c) which illustrates the positions of the detected portions 140ₚ and 140_{d} of the vessel 140 in relation to a fixed reference position.

In the operation S130, motion vectors 150 that represent a motion of the portion 140ₚ, 140_{d} of the vessel, are extracted from the sequence of angiographic images 120, by determining changes in the position of the detected portion 140ₚ, 140_{d} of the vessel in the angiographic images. The motion vectors 150 may for example represent a motion of the portion 140ₚ, 140_{d} of the vessel with respect to a reference image in the sequence. The reference image may be the image illustrated in Fig. 5a at time t = t₀, for example.

In one example, the motion vectors are expressed using a parametric model that describes the motion of the portion 140ₚ, 140_{d} of the vessel over time. The parametric model may express the two-dimensional motion of the portion 140ₚ, 140_{d} of the vessel over time. The parametric model may express the motion as the combination of a translation, rotation, and optionally a stretching of the portion 140ₚ, 140_{d} of the vessel along the axis of the vessel, over time, for example.

Referring now to the operation S140 mentioned above with reference to Fig. 1 and Fig. 2; in this operation, the angiographic images in the sequence 120 are mutually registered based on the extracted motion vectors 150. With reference to Fig. 5, in this operation the motion vector(s) 150 are used to register the images to a common reference image. The images may be registered to the image illustrated in Fig. 5a at time t = to, for example. This results in the images in the temporal sequence being mutually aligned based on the detected portion 140ₚ, 140_{d} of the vessel. The registration that is performed in this operation may be rigid, or alternatively it may be non-rigid, i.e. elastic. A rigid registration may be used in situations in which the vessel 140 undergoes a relatively small amount of deformation between the images in the sequence. However, if the vessel 140 undergoes a significant amount of deformation, an elastic registration may be used. If the vessel 140 undergoes significant deformation, the use of an elastic registration can improve the accuracy of the image representation of the occluded vessel in the subsequent operation S150.

In general, two or more images may be registered in the operation S140. The angiographic images that are registered in the operation S140 correspond to a time period. In general, the time period may be any specified time period. By way of some examples, the images may correspond to a full cardiac cycle, or multiple cardiac cycles, or a portion of a cardiac cycle. In some examples, all of the images in the sequence 120 during a specified time period may be registered in the operation S140. However, in other examples, a selection, i.e. a proper subset of the images from the specified time period may be registered. Some of the motion vectors 150 that are extracted in the operation S130 may be anomalous because they indicate excessive motion, or because they indicate unexpected positions of the portions of the vessel 140ₚ, 140_{d}. This may be due to poor accuracy in detecting the portion of the vessel in the operation S120, or poor accuracy in extracting the motion vectors 150 in the operation S130. In such cases, the operation S140 may be performed for a selection of the images from the temporal sequence, e.g. for a proper subset of the images for which the extracted motion vectors 150 fit within an envelope of expected motion. Omitting such anomalous motion vectors results in a more accurate representation of the occluded vessel in the subsequent operation S150.

Referring now to the operation S150 mentioned above with reference to Fig. 1 and Fig. 2; in this operation, the image intensities in the mutually-registered sequence of angiographic images in a region of interest adjoining the portion 140ₚ, 140_{d} of the vessel, are processed to provide an image representation 160 of the occluded vessel.

In the operation S150, image intensities in the mutually-registered sequence of angiographic images are processed in a region of interest adjoining the portion 140ₚ, 140_{d} of the vessel. In general, this operation may include processing the image intensities only within the region of interest, or it may include processing the image intensities across the entirety of each angiographic image in the sequence, and therefore across a region that inherently includes the region of interest adjoining the portion 140ₚ, 140_{d} of the vessel. The region of interest may be defined in various ways. For example, the region of interest may be defined by a predetermined shape, such as a rectangle, or an oval shape, adjoining the portion(s) 140ₚ, 140_{d} of the vessel. Alternatively, the region of interest may be defined as a portion of the angiographic images encompassing an expected path of the occluded vessel. The region of interest may have a predetermined length along the expected path and/ or a predefined width either side of the expected path of the occluded vessel, for example.

The processing that is performed in the operation S150 may be performed using various techniques. In one example, the one or more processors 110 are configured to process S150 the image intensities in the mutually-registered sequence of angiographic images by temporally integrating the image intensities in the region of interest over a plurality of the mutually-registered images. The temporal integration serves to enhance the intensity of faint vessel features in the angiographic images such as vessel walls, calcifications, and any contrast agent that may be present along the occlusion 130. A consequence of the temporal integration is therefore to provide an image representation of the occluded vessel wherein the visibility of the occlusion is enhanced as compared to its visibility in the sequence of angiographic images 120. A further consequence of the temporal integration is to provide, in the image representation 160 of the occluded vessel, an extension to the portion 140ₚ, 140_{d} of the vessel abutting the occlusion 130 towards the occlusion 130. This extension is a result of the enhancement of the visibility of the occlusion. The temporal integration may also have the beneficial effect of blurring background image features surrounding the occluded vessel. This is because the motion vectors, due to the way they are calculated, correspond to the motion of the occluded vessel, which might move, as in the cardiac case, differently from the background image features. Registering with respect to the occluded vessel has then the effect of producing unaligned background features in the registered sequence, which are therefore blurred and attenuated when temporal integration is applied to that sequence.

The temporal integration of the image intensities may be performed using various techniques. In one example, the temporal integration includes calculating a weighted average of the pixel intensities in the mutually-registered images in the temporal sequence. For example, the temporal integration may include applying weights to the pixel intensities in a selection of the last N images in the temporal sequence, and calculating the pixel intensities in the image representation 160 as an average of the weighted pixel intensities for the corresponding pixels. The weights that are applied to the images may be the same for all pixels, and the same for all N images. Alternatively, the weights may be different for different images and/or they may be different within an image. For instance, different weights may be applied to the images based on an estimated accuracy of the motion vector(s), or an image quality, of the image. Alternatively, different weights may be applied within, and outside, the region of interest for an image, in order to emphasize the importance of features within the region of interest over those outside the region of interest.

In one example, the region of interest includes a calcified vessel segment. Calcified vessel segments are often present in occlusions because vessel calcification is a stage in the development of some types of occlusions. Calcifications have relatively higher X-ray attenuation than vessel tissue, and consequently calcified vessel segments can serve to indicate a path of an occluded vessel in the absence of contrast agent. Thus, if the region of interest includes a calcified vessel segment, the processing operation S150, e.g. temporally integrating the image intensities in the region of interest over a plurality of the mutually-registered images, improves the enhancement of the image intensities within the vessel, and consequently provides a clearer representation of the occluded vessel in the image representation 160.

Instead of temporally integrating the image intensities in the operation S150, other techniques may alternatively be used to provide the image representation 160 of the occluded vessel. For instance, a trained neural network may be used to predict the image representation 160 of the occluded vessel from the mutually-registered angiographic images that are provided by the operation S140. The neural network may be trained using sequences of mutually-registered images of occluded vessels, and for each sequence, a corresponding ground truth image of the vessel in which the occlusion is visible. The ground truth images may be obtained using a different imaging modality to the angiographic images 120 and in which the occlusion is visible. For instance, the ground truth images may include external ultrasound images, or CT images. The image representation 160 that is predicted by the neural network in this example serves to provide an image representation of the occluded vessel wherein the visibility of the occlusion is enhanced as compared to its visibility in the sequence of angiographic images 120.

Referring now to the operation S160 mentioned above with reference to Fig. 1 and Fig. 2; in this operation, the image representation 160 of the occluded vessel is outputted. The image representation may appear similar to the angiographic image 120 illustrated in Fig. 3, additionally including image features representing the occlusion 130. The image representation may be referred to as an enhanced image representation of the occluded vessel since the visibility of the occluded vessel is enhanced as compared to in the sequence of angiographic images.

The image representation 160 may be outputted in various ways, including to a display device such as to the monitor 220 illustrated in Fig. 1, or to a virtual/ augmented reality display device, or to a printer, or to a computer-readable storage medium, and so forth. In general, the image representation 160 may be provided in the form of a single image, or it may be provided in the form of a video.

An image representation 160 in the form of a single image may be generated from a sequence of two or more angiographic images 120 by performing the operations of detecting S120, extracting S130, mutually registering S140, and processing S150, on the images in the sequence, and then outputting S160 a single image representation 160 for the images in the sequence.

Alternatively, an image representation 160 in the form of a video may be generated by outputting the image representation 160 for a specified number (e.g. k ≥ 2) of angiographic images 120, and then updating the image representation 160 as additional images from the sequence of angiographic images 120 are taken into account. For example, the image representation 160 may be generated for a specified number (e.g. k ≥ 2) of angiographic images 120 by performing the operations of detecting S120, extracting S130, mutually registering S140, and processing S150, on all k images. The image representation 160 for the k images may then be outputted. The image representation 160 may then be updated each time an additional image from the sequence is taken into account by performing the operations of detecting S120, extracting S130, mutually registering S140, and processing, using the additional image. In this example, the visibility of the occlusion in the video image representation 160 improves as each additional image is taken into account. Moreover, the portion 140ₚ, 140_{d} of the vessel abutting the occlusion 130 extends towards the occlusion 130 as each subsequent image is taken into account as a result of the improvement of its visibility.

In one example, the one or more processors 110 are configured to predict an extension to the portion 140ₚ, 140_{d} of the vessel abutting the occlusion 130 towards the occlusion 130. In this example, the image representation 160 comprises the predicted extension. The extension in this example may be predicted using various techniques. These include the local minimal path technique described in a document by Benmansour, F., et al., "Fast object segmentation by growing minimal paths from a single point on 2D or 3D images. J Math Imaging Vis 33, 209-221, 2009, https://doi.org/10.1007/s10851-008-0131-0. As another example, a minimal path technique such as Fast Marching may be used to predict the extension. Such minimal path techniques try to find a path that minimizes a value of a cost function. Alternatively, the extension in this example may be predicted using a neural network, such as the nnU-Net described in the document by Isensee, F., et al., cited above.

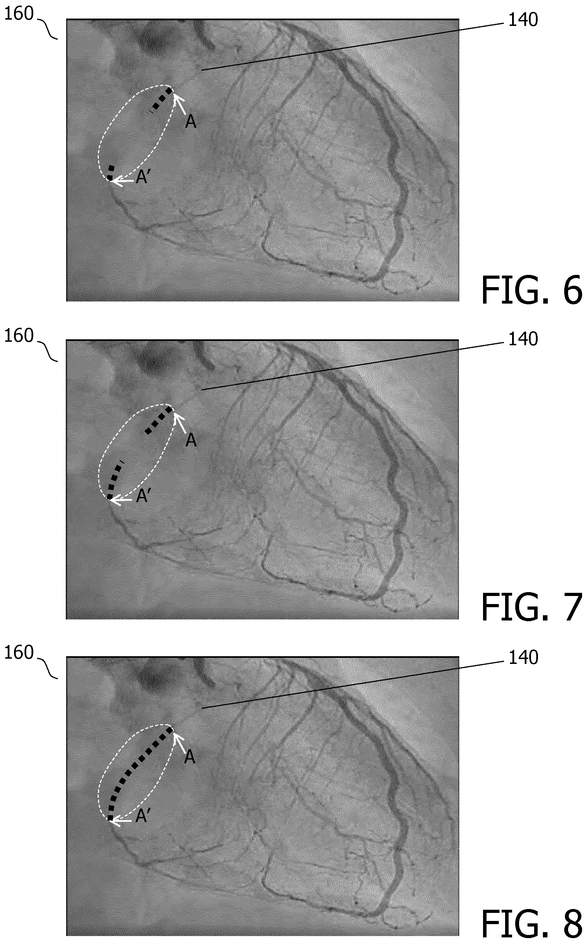

Various examples of an image representation 160 of an occluded vessel that include such an extension to the portion of the vessel abutting the occlusion are illustrated in Fig. 6 - Fig. 8. The illustrations in Fig. 6 - Fig. 8 represent the extension by way of the dotted line in each image.

In one example, a single extension is predicted from a plurality of angiographic images. This example is illustrated in Fig. 6, which is a first example of an image representation 160 of an occluded vessel including an extension to the portion of the vessel abutting the occlusion towards the occlusion, in accordance with some aspects of the present disclosure. In this example, the operations S120, S130, S140 and S150 are performed on the plurality of angiographic images, such as the two angiographic images illustrated in Fig. 5a) and 5b). This results in an image representation 160 of the occluded vessel. The image representation 160 of the occluded vessel may be generated by temporally integrating the image intensities in a region of interest over the two mutually-registered images in the operation S150. The temporal integration provides a clearer representation of the occluded vessel than in the individual angiographic images illustrated in Fig. 5a) and Fig. 5b). The extension illustrated in Fig. 6 is then predicted from the image representation 160 of the occluded vessel using the minimal path techniques described above. This extension is then outputted in the image representation 160 of the occluded vessel, for example in the form of a dotted line as illustrated in Fig. 6.

In the technique described above with reference to Fig. 6, a single extension may be predicted using the result of a temporal integration operation in which any number of angiographic images from the sequence are taken into account. In general, the clarity of the image representation 160 of the occluded vessel increases with the number of angiographic images that are used in the temporal integration operation. However, the occluded vessel can deform throughout the sequence of angiographic images. This deformation can limit the reliability of the temporal integration operation because it tends to blur the occluded vessel in the image representation 160. In-turn, the vessel deformation can limit the reliability of an extension that is predicted based on the result of the temporal integration. In one example, the effects of such vessel deformation are mitigated by predicting the extension iteratively. In this example, the one or more processors 110 are configured to perform the extracting S130, the mutually registering S140, the processing S150, and the predicting, iteratively, and wherein each iteration uses the predicted extension to the portion 140ₚ, 140_{d} of the vessel from a previous iteration as the detected portion of the vessel in the extracting S130.

The effect of performing the above operations iteratively is to provide a more reliable position of the portion of the vessel that is used as the starting point for each temporal integration. This in-turn improves the reliability of the predicted extension, and so on.

The result of a single iteration of the above example corresponds to the single extension described above. An example of the image representation that is provided after two iterations of the above operations is illustrated in Fig. 7, which is a second example of an image representation 160 of an occluded vessel including an extension to the portion of the vessel abutting the occlusion towards the occlusion, in accordance with some aspects of the present disclosure. An example of the image representation that is provided after three iterations of the above operations is illustrated in Fig. 8, which is a third example of an image representation 160 of an occluded vessel including an extension to the portion of the vessel abutting the occlusion towards the occlusion, in accordance with some aspects of the present disclosure. The extension illustrated in Fig. 8 illustrates the complete length of the occlusion 130, i.e. the complete length of the occlusion between the points labelled A, and A', in the vessel 130 illustrated in Fig. 3. As may be appreciated from the illustrations in Fig. 6 - Fig. 8, in this example, the length of the predicted extension, and also the clarity of the occlusion, both increase in each iteration.

In the operation S160, the image representations 160 illustrated in Fig. 6 - Fig. 8 may be outputted in the form of a video. The video illustrates an incremental increase in the predicted extension to the vessel in each iteration. Outputting the images illustrated in Fig. 6 - Fig. 8 in the form of a video in which the extension grows in each iteration facilitates a user to observe the development of the extension. Instead of outputting the image representations 160 illustrated in Fig. 6 - Fig. 8 in the form of a video, a single image representations 160, for example the image representation 160 that represents a final iteration illustrated in Fig. 8, or an intermediate iteration, in the iterative process, may be outputted in the operation S160.

As mentioned above, in general, a single, i.e. a proximal or a distal, or alternatively both, i.e. a proximal and a distal, portion of the vessel 140 abutting the occlusion 130 may be detected in the operation S120. With reference to the latter situation, in one example, the system described above with reference to Fig. 1 is used to detect S120 a proximal portion 140ₚ of the vessel abutting the occlusion 130, and to extract S130 motion vectors 150 representing a motion of the proximal portion 140ₚ of the vessel. In this example, the one or more processors 110 are also configured to:
detect a distal portion 140_{d} of the vessel 140 abutting the occlusion 130 in the sequence of angiographic images 120;
extract, from the sequence of angiographic images 120, motion vectors representing a motion of the distal portion 140_{d} of the vessel, based on the detected distal portion of the vessel;
and wherein the one or more processors 110 are configured to mutually register S140 the sequence of angiographic images 120 based further on the extracted motion vectors representing the motion of the distal portion 140_{d} of the vessel.

In this example, the detection of the distal portion 140_{d} of the vessel, and the extraction of motion vectors representing a motion of the distal portion 140_{d} of the vessel, may be performed in the same manner as described above for the proximal portion 140ₚ. Using both the proximal and the distal portion of the vessel in accordance with this example may serve to improve the reliability of the image representation of the vessel because the path of the vessel is constrained at both ends by the detected portions of the vessel.

In one example, the image representation 160 of the occluded vessel is registered to a live sequence of fluoroscopic images. In this example, the one or more processors 110 are configured to:
receive fluoroscopic image data representing a live sequence of fluoroscopic images comprising the occlusion 130 in the vessel 140;
register the image representation 160 of the occluded vessel to the live sequence of fluoroscopic images; and
wherein the one or more processors 110 are configured to output the live sequence of fluoroscopic images and wherein one or more of:
   the image representation 160 and the live sequence of fluoroscopic images are displayed as an overlay image;
   the image representation 160 and the live sequence of fluoroscopic images are displayed adjacent one another;
   a path of the occluded vessel 140 is extracted from the image representation 160 and displayed in the live sequence of fluoroscopic images;
   one or more features of the occluded vessel 140 are extracted from the image representation 160 and displayed in the live sequence of fluoroscopic images.

Live sequences of fluoroscopic images are often acquired during treatment procedures in order to navigate interventional devices to an occlusion. Fluoroscopic images indicate radiopaque materials such as interventional devices, and also dense portions of the anatomy such as bone. However, the vasculature, and also soft tissue, are poorly represented in fluoroscopic images. In order to visualize the vasculature, a contrast agent may be intermittently injected into the vasculature during a fluoroscopic imaging procedure. However, the use of contrast agent incurs health risks, and so it is desirable to reduce the use of contrast agent during fluoroscopic imaging. Besides, in case of occlusions, the use of intermittently injected contrast agent is completely ineffective.

In this example, the registration of the image representation 160 of the occluded vessel to the live sequence of fluoroscopic images provides spatial correspondence between the occluded vessel and the fluoroscopic images. The registration may be performed in accordance with a technique used in the Dynamic Coronary Roadmapping product marketed by Philips Healthcare, Best, The Netherlands, and which is described in the document WO 2008/104921 A2. Improved guidance is provided to a physician by each of: displaying the image representation 160 and the live sequence of fluoroscopic images as an overlay image; displaying the image representation 160 and the live sequence of fluoroscopic images adjacent one another; displaying a path of the occluded vessel 140 in the live sequence of fluoroscopic images; and displaying one or more features of the occluded vessel 140 in the live sequence of fluoroscopic images. Moreover, this additional guidance is provided without the need for additional contrast agent injections. Thus, it also reduces the health risks associated with contrast agent usage.

In this example, in the overlay image, the image representation 160 of the occluded vessel may be distinguished from the live sequence of fluoroscopic images by using various colors, or by using various markers, to highlight the occluded vessel.

In this example, the one or more features of the occluded vessel 140 that are extracted from the image representation 160 may include features such as calcifications, for example. Calcifications are visible in the image representation by virtue of their relatively higher X-ray attenuation values as compared to blood vessels. Calcification features may be extracted from the image representation 160 using segmentation techniques such as those mentioned above.

In a related example, the live sequence of fluoroscopic images comprises an interventional device in the vessel 140. The one or more processors 110 are configured to:
detect a portion of the interventional device in the live sequence of fluoroscopic images;
extract, from the live sequence of fluoroscopic images, motion vectors representing a motion of the portion of the interventional device, based on the detected portion of the interventional device; and
wherein the one or more processors 110 are configured to mutually register S140 the sequence of angiographic images 120 based further on the extracted motion vectors representing the motion of the portion of the interventional device.

In this example, the motion of the portion of the interventional device, as determined from the fluoroscopic images, is used in combination with the motion of the portion 140ₚ, 140_{d} of the vessel abutting the occlusion 130, as determined from the angiographic images, to mutually register the angiographic images. The portion of the interventional device that is detected in this example may for instance be a radiopaque tip of the interventional device, or a radiopaque marker attached to the interventional device, a portion of a guidewire formed from a radiopaque material, and so forth. The radiopacity of such interventional device features facilitates their detection in the fluoroscopic images in the absence of a contrast agent. Thus, the portion of the interventional device serves as an additional feature to mutually register the angiographic images. The portion of the interventional device serves as a reliable feature to mutually register the angiographic images because the interventional device is within the same occluded vessel in both the angiographic images and the fluoroscopic images, and consequently the motion of the interventional device that is determined from the fluoroscopic images, is similar to the motion of the vessel that is determined from the detected portion of the vessel in the angiographic images. Another benefit of using the portion of the interventional device to mutually register the angiographic images is that it does not require contrast agent to make it visible in the fluoroscopic images. A further benefit of using the interventional device to perform this registration is that as the interventional device advances into the occlusion in the fluoroscopic images, it follows the path of the occlusion. Consequently, the motion of the interventional device, as determined from the fluoroscopic images, provides reliable information on the motion of the occluded vessel, and also the shape, of the occluded vessel, within the occlusion itself. Thus, it serves to improve the reliability of the image representation 160 of the occluded vessel.

In another example, a volumetric angiographic image comprising the vessel 140 is used to estimate a shape of the vessel 140 in the sequence of angiographic images 120. In this example, the image data that is received in the operation S110 comprises projection image data and the sequence of angiographic images comprises a sequence of projected angiographic images, and the one or more processors 110 are also configured to:
receive volumetric image data representing a volumetric angiographic image comprising the vessel 140; and
estimate a shape of the vessel 140 in the sequence of angiographic images 120 based on the volumetric angiographic image; and
wherein one or more processors 110 are configured to detect S120 the portion 140ₚ, 140_{d} of the vessel abutting the occlusion 130 in the sequence of angiographic images 120 based on the estimated shape of the vessel 140.

In this example, the volumetric angiographic image is used to estimate a shape of the vessel 140 in the angiographic images 120, and the estimated shape of the vessel 140 in the angiographic images 120 is used to detect S120 the portion 140ₚ, 140_{d} of the vessel abutting the occlusion 130 in the sequence of angiographic images 120.. This may improve the reliability of the detection of the portion 140ₚ, 140_{d} of the vessel, or it may reduce the time taken to detect the portion 140ₚ, 140_{d} of the vessel because the volumetric angiographic image, in contrast to the projection angiographic images that are received in the operation S110, includes additional information on the shape and movement of the portion of the vessel 140. Consequently, it improves the reliability of the image representation 160 of the occluded vessel, and also reduces the time taken to generate the image representation 160.

In this example, the shape of the vessel 140 in the sequence of angiographic images may be estimated by identifying the vessel 140 in the volumetric angiographic image, and subsequently registering the volumetric angiographic image, including the identified vessel, to the sequence of projection angiographic images that are acquired in the operation S110. The portion 140ₚ, 140_{d} of the vessel abutting the occlusion 130 in the sequence of angiographic images 120 may be determined by finding a (minimal) path in the angiographic images path that is constrained by the projected estimated shape of the vessel. The vessel 140 may be identified in the volumetric angiographic image using segmentation techniques such as model-based segmentation, semantic segmentation, and so forth. The registration may be performed by projecting the volumetric angiographic image, including the identified vessel, so as to provide a synthetic projection image from the same perspective of a projection X-ray imaging system as the sequence of projection angiographic images, and then (elastically) registering the synthetic projection image to the projection angiographic images. This provides synthetic projection images, which include the identified vessel 140, and which correspond to the projection angiographic images, and consequently it facilitates the identification of the identified vessel in the projection angiographic images.

In this example, the volumetric angiographic image may be a (pre-procedural) CT image or a (pre-procedural) MRI image, for example. In the former case, the volumetric angiographic image may be referred-to as a coronary computed tomography angiography "CCTA" image if the vessel is in the heart.

In another example, a volumetric angiographic image comprising the vessel 140 is registered to the angiographic images. In this example, the image data that is received in the operation 5110 comprises projection image data and the sequence of angiographic images comprises a sequence of projected angiographic images, and the one or more processors 110 are also configured to:
receive volumetric image data representing a volumetric angiographic image comprising the vessel 140;
elastically register the volumetric angiographic image to the projected angiographic images based on the extracted motion vectors; and
wherein the image representation 160 of the occluded vessel comprises the volumetric angiographic image elastically registered to the occluded vessel.

In this example, the motion vectors may be extracted in the manner described above.

In another example, the one or more processors 110 are configured to evaluate an accuracy parameter for the image representation 160 and to output the value of the accuracy parameter. The value of the accuracy parameter may be calculated based on factors such as a sharpness of the image representation 160 within the region of interest, or based on a confidence measure for the operation of detecting (S120) the portion of the vessel abutting the occlusion. Alternatively, or additionally, the value of the accuracy parameter may be calculated using the motion vectors. For instance, if the motion field has an excessive magnitude, or if the motion field is indicative of a high degree of bending, a relatively lower accuracy parameter may be provided. Providing the value of the accuracy parameter allows a physician to assess the extent to which the image representation 160 can be relied upon.

In a related example, an estimated extension to the portion of the vessel 140ₚ, 140_{d} is provided. In this example, in response to the value of the accuracy parameter being below a predetermined threshold value, the one or more processors 110 are configured to:
estimate an extension to the portion of the vessel 140ₚ, 140_{d} abutting the occlusion 130, the extension being towards the occlusion; and
output the estimated extension to the portion of the vessel 140ₚ, 140_{d} in the image representation 160 of the occluded vessel.

In this example, the estimated extension may be determined techniques such as interpolation (e.g. using a spline curve), or a weighted least-square regression of a parametric shape, where the weights are determined based on the values of the accuracy parameters.

In another example, the value of the accuracy parameter may be indicated in the image representation. For instance, the region of interest adjoining the portion of the vessel, or the predicted extension to the vessel, or the estimated extension to the vessel, may be provided with a color that corresponds to the value of the accuracy parameter. This indicates to a physician the extent to which the image representation 160 can be relied upon.

In another example, the sequence of angiographic images 120 comprises an interventional device in the vessel 140, and the one or more processors 110 are configured to:
detect a portion of the interventional device in the sequence of angiographic images 120;
extract, from the sequence of angiographic images 120, motion vectors representing a motion of the portion of the interventional device, based on the detected portion of the interventional device; and
wherein the one or more processors 110 are configured to mutually register S140 the sequence of angiographic images 120 based further on the extracted motion vectors representing the motion of the portion of the interventional device.

In this example, the portion of the interventional device that is detected may for instance be a radiopaque tip of the interventional device, or a radiopaque marker attached to the interventional device, a portion of a guidewire formed from a radiopaque material, and so forth. The radiopacity of such features of the interventional device facilitate their detection in the angiographic images. The detected portion of the interventional device provides a reliable landmark that is used in addition to the detected portion 140ₚ, 140_{d} of the vessel abutting the occlusion 130, to mutually register S140 the sequence of angiographic images 120. The use of this additional landmark improves the reliability of the registration, and consequently it improves the reliability of the image representation 160 of the occluded vessel. An additional benefit is provided if the portion of the interventional device is in contact with the occlusion because in this arrangement the detected portion of the interventional device provides an accurate indication of the vessel motion close to the occlusion, i.e. at a point in the vessel in which the visibility of the portion 140ₚ, 140_{d} of the vessel abutting the occlusion 130 starts to diminish as a result of the occlusion.

It is noted that in the examples described above, the system 100 may also include one or more of: an angiographic imaging system for providing the image data that is received in the operation S110, or for providing the fluoroscopic image data, such as for example the projection X-ray imaging system 210 illustrated in Fig. 1; a display device such as the monitor 220 illustrated in Fig. 1 for displaying the image representation 160 of the occluded vessel, fluoroscopic images, and other data outputted by the one or more processors 110 of the system 100; a patient bed 230; an injector (not illustrated in Fig. 1) for injecting a contrast agent into a subject; and a user input device (not illustrated in Fig. 1) for receiving user input in connection with the system 100, such as a keyboard, a mouse, a touchscreen, and so forth.

In another example, an angiographic imaging system that includes the system 100, is provided.

In another example, a computer-implemented method of providing an image representation of an occluded vessel, is provided. The method includes:
receiving S110 image data representing a sequence of angiographic images 120 comprising an occlusion 130 in a vessel 140;
detecting S120 a portion 140ₚ, 140_{d} of the vessel abutting the occlusion 130 in the sequence of angiographic images 120;
extracting S130, from the sequence of angiographic images 120, motion vectors 150 representing a motion of the portion 140ₚ, 140_{d} of the vessel, based on the detected portion of the vessel;
mutually registering S140 the sequence of angiographic images 120 based on the extracted motion vectors 150;
processing S150 the image intensities in the mutually-registered sequence of angiographic images in a region of interest adjoining the portion 140ₚ, 140_{d} of the vessel to provide an image representation 160 of the occluded vessel; and
outputting S160 the image representation 160 of the occluded vessel.

In another example, a computer program product, is provided. The computer program product comprises instructions which when executed by one or more processors 110, cause the one or more processors to carry out a method of providing an image representation of an occluded vessel. The method includes:
receiving S110 image data representing a sequence of angiographic images 120 comprising an occlusion 130 in a vessel 140;
detecting S120 a portion 140ₚ, 140_{d} of the vessel abutting the occlusion 130 in the sequence of angiographic images 120;
extracting S130, from the sequence of angiographic images 120, motion vectors 150 representing a motion of the portion 140ₚ, 140_{d} of the vessel, based on the detected portion of the vessel;
mutually registering S140 the sequence of angiographic images 120 based on the extracted motion vectors 150;
processing S150 the image intensities in the mutually-registered sequence of angiographic images in a region of interest adjoining the portion 140ₚ, 140_{d} of the vessel to provide an image representation 160 of the occluded vessel; and
outputting S160 the image representation 160 of the occluded vessel.

The above examples are to be understood as illustrative of the present disclosure, and not restrictive. Further examples are also contemplated. For instance, the examples described in relation to a system, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing an image representation of an occluded vessel, the system comprising one or more processors (110) configured to:
receive (S110) image data representing a sequence of angiographic images (120) comprising an occlusion (130) in a vessel (140);
detect (S120) a portion (140ₚ, 140_{d}) of the vessel abutting the occlusion (130) in the sequence of angiographic images (120);
extract (S130), from the sequence of angiographic images (120), motion vectors (150) representing a motion of the portion (140ₚ, 140_{d}) of the vessel, based on the detected portion of the vessel;
mutually register (S140) the sequence of angiographic images (120) based on the extracted motion vectors (150);
process (S150) the image intensities in the mutually-registered sequence of angiographic images in a region of interest adjoining the portion (140ₚ, 140_{d}) of the vessel to provide the image representation (160) of the occluded vessel; and
output (S160) the image representation (160) of the occluded vessel.

2. The system according to claim 1, wherein the one or more processors (110) are configured to process (S150) the image intensities in the mutually-registered sequence of angiographic images by temporally integrating the image intensities in the region of interest over a plurality of the mutually-registered images.

3. The system according to claim 2, wherein the one or more processors (110) are further configured to predict an extension to the portion (140ₚ, 140_{d}) of the vessel abutting the occlusion (130) towards the occlusion (130), and wherein the image representation (160) comprises the predicted extension.

4. The system according to claim 3, wherein the one or more processors (110) are configured to perform the extracting (S130), the mutually registering (S140), the processing (S150), and the predicting, iteratively, and wherein each iteration uses the predicted extension to the portion (140ₚ, 140_{d}) of the vessel from a previous iteration as the detected portion of the vessel in the extracting (S130).

5. The system according to any previous claim, wherein the portion of the vessel (140) comprises a proximal portion (140ₚ), and wherein the one or more processors (110) are further configured to:
detect a distal portion (140_{d}) of the vessel (140) abutting the occlusion (130) in the sequence of angiographic images (120);
extract, from the sequence of angiographic images (120), motion vectors representing a motion of the distal portion (140_{d}) of the vessel, based on the detected distal portion of the vessel;
and wherein the one or more processors (110) are configured to mutually register (S140) the sequence of angiographic images (120) based further on the extracted motion vectors representing the motion of the distal portion (140_{d}) of the vessel.

6. The system according to any previous claim, wherein the one or more processors (110) are further configured to:
receive fluoroscopic image data representing a live sequence of fluoroscopic images comprising the occlusion (130) in the vessel (140);
register the image representation (160) of the occluded vessel to the live sequence of fluoroscopic images; and
wherein the one or more processors (110) are further configured to output the live sequence of fluoroscopic images and wherein one or more of:
the image representation (160) and the live sequence of fluoroscopic images are displayed as an overlay image;
the image representation (160) and the live sequence of fluoroscopic images are displayed adjacent one another;
a path of the occluded vessel (140) is extracted from the image representation (160) and displayed in the live sequence of fluoroscopic images;
one or more features of the occluded vessel (140) are extracted from the image representation (160) and displayed in the live sequence of fluoroscopic images.

7. The system according to claim 6, wherein the live sequence of fluoroscopic images comprises an interventional device in the vessel (140), and wherein the one or more processors (110) are further configured to:
detect a portion of the interventional device in the live sequence of fluoroscopic images;
extract, from the live sequence of fluoroscopic images, motion vectors representing a motion of the portion of the interventional device, based on the detected portion of the interventional device; and
wherein the one or more processors (110) are configured to mutually register (S140) the sequence of angiographic images (120) based further on the extracted motion vectors representing the motion of the portion of the interventional device.

8. The system according to any previous claim, wherein the received image data comprises projection image data and the sequence of angiographic images comprises a sequence of projected angiographic images, and wherein the one or more processors (110) are further configured to:
receive volumetric image data representing a volumetric angiographic image comprising the vessel (140); and
estimate a shape of the vessel (140) in the sequence of angiographic images (120) based on the volumetric angiographic image; and
wherein one or more processors (110) are configured to detect (S120) the portion (140ₚ, 140_{d}) of the vessel abutting the occlusion (130) in the sequence of angiographic images (120) based on the estimated shape of the vessel (140).

9. The system according to any previous claim, wherein the one or more processors (110) are further configured to:
evaluate an accuracy parameter for the image representation (160); and
output the value of the accuracy parameter.

10. The system according to claim 9, wherein in response to the value of the accuracy parameter being below a predetermined threshold value, the one or more processors (110) are further configured to:
estimate an extension to the portion of the vessel (140ₚ, 140_{d}) abutting the occlusion (130), the extension being towards the occlusion; and
output the estimated extension to the portion of the vessel (140ₚ, 140_{d}) in the image representation (160) of the occluded vessel.

11. The system according to any previous claim, wherein the sequence of angiographic images (120) further comprises an interventional device in the vessel (140), and wherein the one or more processors (110) are further configured to:
detect a portion of the interventional device in the sequence of angiographic images (120);
extract, from the sequence of angiographic images (120), motion vectors representing a motion of the portion of the interventional device, based on the detected portion of the interventional device; and
wherein the one or more processors (110) are configured to mutually register (S140) the sequence of angiographic images (120) based further on the extracted motion vectors representing the motion of the portion of the interventional device.

12. The system according to any previous claim, wherein the region of interest comprises a calcified vessel segment.

13. The system according to any previous claim, wherein the image representation (160) of the occluded vessel comprises an extension to the portion (140ₚ, 140_{d}) of the vessel abutting the occlusion (130) towards the occlusion (130).

14. A computer-implemented method of providing an image representation of an occluded vessel, the method comprising:
receiving (S110) image data representing a sequence of angiographic images (120) comprising an occlusion (130) in a vessel (140);
detecting (S120) a portion (140ₚ, 140_{d}) of the vessel abutting the occlusion (130) in the sequence of angiographic images (120);
extracting (S130), from the sequence of angiographic images (120), motion vectors (150) representing a motion of the portion (140ₚ, 140_{d}) of the vessel, based on the detected portion of the vessel;
mutually registering (S140) the sequence of angiographic images (120) based on the extracted motion vectors (150);
processing (S150) the image intensities in the mutually-registered sequence of angiographic images in a region of interest adjoining the portion (140ₚ, 140_{d}) of the vessel to provide an image representation (160) of the occluded vessel; and
outputting (S160) the image representation (160) of the occluded vessel.

15. A computer program product comprising instructions which when executed by one or more processors (110), cause the one or more processors to carry out the method according to claim 14.
